# EUROPEAN PATENT SPECIFICATION

(11) **EP 4 374 807 B1**
(45) Date of publication and mention of the grant of the patent: **12.11.2025**
(21) Application number: 23211644.2
(22) Date of filing: 23.11.2023
(51) Int. Cl.: A61B 17/64

(54) **CLAMP FOR EXTERNAL FIXATION DEVICE**
KLEMME FÜR EINE EXTERNE FIXIERVORRICHTUNG
PINCE DE DISPOSITIF DE FIXATION EXTERNE

(30) Priority: 25.11.2022 IT 202200024405
(43) Date of publication of application: 29.05.2024
(73) Proprietor: Citieffe S.r.L., 40012 Calderara Di Reno (BO) (IT)
(72) Inventor: DOVESI, Alan, Bologna (IT); MORETTI, Massimiliano, Calderara di Reno (Bologna) (IT)
(74) Representative: Firmati, Leonardo

(56) References cited:
- CN-A- 112 932 636
- GB-A- 2 110 094
- US-A- 5 160 335
- US-A1- 2012 209 266

## Description

This invention relates to external orthopaedic fixation devices.

In particular, the invention relates to a clamp of an orthopaedic external fixation device.

In recent years, in orthopaedics, a technique has been widespread for stabilising fractures, especially in the long bones of the limbs, which does not need to make use of traditional plaster casts, using the so-called external fixation devices instead.

External fixation devices usually comprise a plurality of bone screws which are implanted in the bone stumps of the fracture in such a way that the head ends of the screws project from the skin of the patient. The ends of the bone screws are anchored to a rigid external frame which is fitted with bars, clamps and joints which can be oriented so as to be adapted to the position of the screws. In the most widespread versions, the bars making up the frame have a cylindrical cross-section and are positioned longitudinally.

The screws usually have a cylindrical body, having, on one side, a threaded portion intended to be screwed into the bone stump and, on the other side, the above-mentioned head end, which is shaped to be able to be coupled to a grip which allows the screw to be screwed into the bone stump. The connection between the screw and the grip is normally of the male-female type with quick coupling and release.

Operatively, after making an incision in the soft tissue, the surgeon makes holes in the bone stumps on opposite sides of the fracture fissure and implants the screws in the holes.

The surgeon then couples the screws, without the temporary grip attached, to the respective clamps of the frame; then, where necessary and possible, the edges of the fracture are aligned in such a way as to position the bone stumps in the most suitable position to knit together.

Once the fracture has been reduced or in any case the desired relative position between the bone stumps has been reached, the surgeon connects and locks the joints and clamps using the above-mentioned longitudinal bars, to keep the bone stumps in the predetermined position, thus allowing the correct formation between the bone stumps of "bone callus", which progressively restores the lamellar bone tissue with which the bone recovers its original continuity and functionality.

The external fixation devices allow, with a hold on the bone far from the fracture centre and with a stabilisation and adjustability outside of the fracture, action to be taken on the bone stumps whilst at the same time leaving the fracture zone free for surface medication.

At the same time, external fixation devices do not interfere with the aeration of the fractured part and reduce the loss of muscle tone normally encountered with the use of plaster casts.

External fixation devices are used not only for fractures caused by impact or falls.

For some diseases, treatment of the long bones of the limbs is in fact required by means of osteotomy, with the formation of two or more bone stumps or segments and subsequent relative movement of these stumps or segments.

These treatments include the lengthening of a limb and the correction of anatomical deformities.

The lengthening of a limb may be necessary for a number of reasons, not only, for example, for aesthetic reasons related to an increase in height. Unlike what one might think, in fact, the majority of bone lengthening treatments are due to diseases that necessitated the removal of portions, sometimes quite large portions, of bone.

Also frequent are cases of deformation of long bones, for example of the humerus, which often constitute a serious impediment to the mobility and autonomy of the patient.

Particularly in the paediatric field, that is, by exploiting the physiological growth of the patient, attempts are made to correct these deformations and the use of an external fixation device has proved to be quite an adequate tool.

Despite this, in some circumstances, for example in the cases of deformation of the humerus with the presence of marked curvature, the existing fixation devices have also not been particularly effective and in fact have been seen to be not free from drawbacks.

One of these drawbacks is linked to the need which often occurs of modifying, even after application of the fixation device, the relative curvature of the two bone stumps.

This factor, requiring modification of the relative orientation in space of the screws fixed to two different bone stumps, also implies the loosening of the respective locking clamps from the connecting bars or also from the screws, thereby adversely affecting the correct relative positioning of the clamps and also making their reciprocal movement rather complex.

A further drawback of the prior art fixation devices, such as, for example, those described in the prior art documents CN 112 932 636, US 5 160 335, US 2012/209266, GB 2 110 094, is referred to the not always certain effectiveness of the devices for fixing movable parts to each other.

The aim of this invention is to provide a clamp for an external fixation device for treating bone fractures which is able to overcome the drawbacks of the prior art and which is at once simple and inexpensive to make and practical to use.

A further aim is to provide a clamp for an external fixation device which allows an effective handling by the operator, also allowing extreme versatility of use. The technical features of the invention, according to the above-mentioned aims, are clearly described in the appended claims and its advantages are apparent from the detailed description which follows, with reference to the accompanying drawings which illustrate a nonlimiting example embodiment of it, and in which:
- Figure 1 is a schematic perspective view from above of a bone fixation device integrating a clamp for the fixation device according to this invention shown in a relative first configuration of use;
- Figure 2 is a schematic perspective view from above of the fixation device of Figure 1 with the clamp according to this invention in a different configuration of use;
- Figure 3 is a schematic perspective view of the clamp according to this invention in its configuration shown in Figure 2;
- Figure 4 is a schematic plan view from above of the clamp of Figure 3;
- Figure 5 is a schematic front elevation view of the clamp of Figure 3;
- Figure 6 is an exploded perspective view of the clamp of Figures 3 to 5;
- Figures 7 and 8 show perspective views from different angles, respectively, of the clamp of Figures 3 to 6, with some parts in cross-section to better illustrate others;
- Figure 9 is an exploded front view of the clamp of Figures 3 to 6.

With reference to the accompanying drawings, the numeral 1 denotes in its entirety an external fixation device for the treatment of bones of the limbs.

The external fixation device 1 comprises a plurality of clamps 2, 3 supporting bone screws 4 to be implanted in respective bone segments not illustrated.

The external fixation device 1 comprises longitudinal connecting bars 5, 6.

The connecting bars 5, 6 extend longitudinally parallel to a predetermined direction D1.

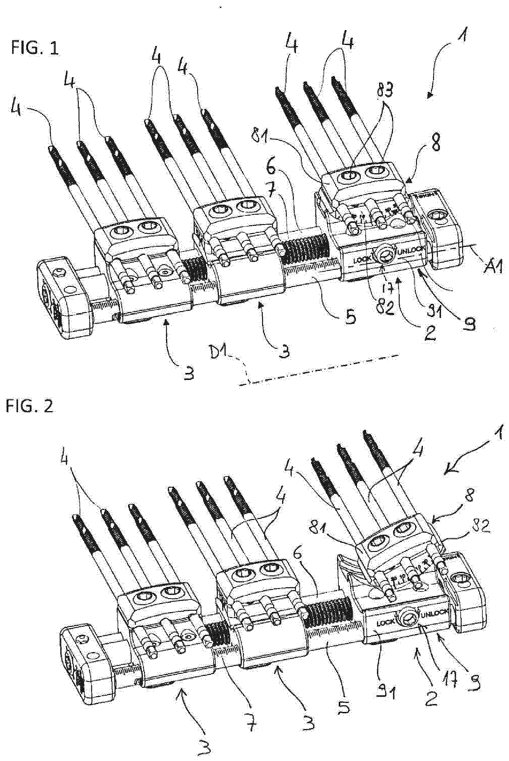

The embodiment of the fixation device 1 illustrated in Figures 1 and 2 also comprises, in the connection between the clamps 2, 3, a threaded bar 7 for controlling the relative movement of the clamps 2, 3; the relative operating methods do not however fall within the context of this invention and will not, therefore, be described further.

As illustrated in Figures 1 and 2, the clamps 2, 3 are designed to stably connect, in the context of an external fixation device 1, the respective bone screws 4 to the longitudinal connecting bars 5, 6.

The clamps 3 are of substantially known types and are not therefore described in detail herein.

The clamp 2 is made according to this invention and comprises a first locking unit 8 designed to house and lock a plurality of bone screws 4 and a second locking unit 9 designed to lock the connecting bars 5, 6, to prevent reciprocal movement between the clamp 2 and the connecting bars 5, 6.

The first locking unit 8 has two valve parts 81, 82, respectively upper and lower, designed to couple to clamp together the above-mentioned bone screws 4 housed in respective housings 10.

The first locking unit 8 also comprises two screws 83 designed to grip together the two upper and lower valve parts 81, 82.

The second locking unit 9 has a main body 91, inside of which suitable through holes 92, 93, 94 are made for slidably housing the connecting bars 5, 6 and the threaded bar 7.

The second locking unit 9 also comprises screw clamping means 94, of substantially known type and not described further, designed both to removably guarantee that the clamp 2 and the connecting bars 5, 6 remain in a reciprocal position, and to activate the engagement and disengagement of the clamp 2 with the threaded bar 7 for the movement of the clamp 2 relative to the connecting bars 6, 7.

The through holes 92, 93 have respective longitudinal axes of extension A1, A2 parallel to the above-mentioned predetermined direction D1.

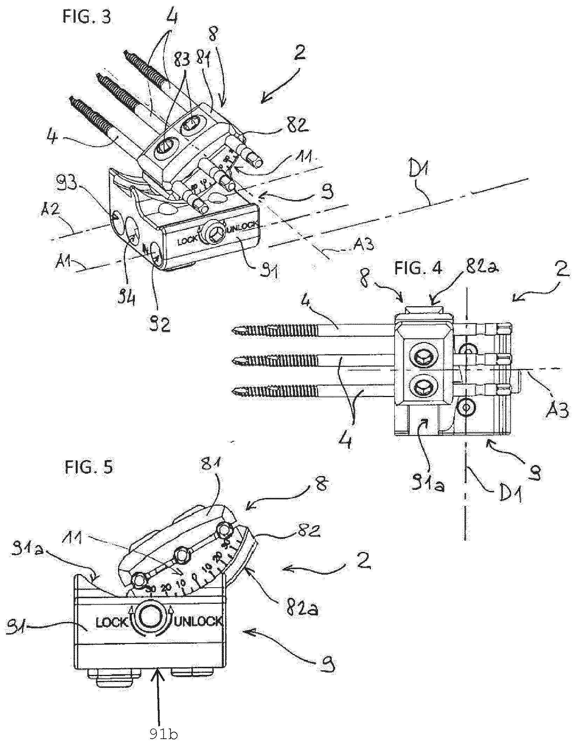

As illustrated in Figures 2 and 3, the first locking unit 8 is mounted like a unit on the main body 91 of the second locking unit 9.

With reference to the accompanying drawings, the lower valve part 82 of the first locking unit 8 has a lower convex curved face 82a.

The main body 91 of the second locking unit 9 also has an upper concave curved wall 91a.

The lower convex curved face 82a of the lower valve part 82 and the upper concave curved wall 91a of the main body 91 are shaped to match each other to engage slidably in a tilting fashion relative to an axis A3 perpendicular to the above-mentioned direction D1.

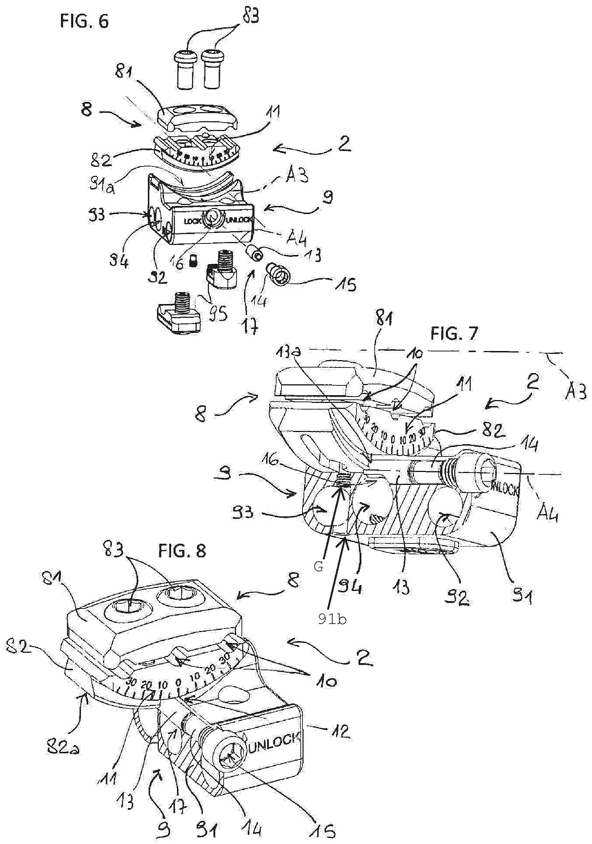

Advantageously, as clearly illustrated in Figures 7 and 8, where the main body 91 has been suitably cut, the above-mentioned convex lower curved face 82a of the lower valve part 82 has the shape of a dovetail.

The dovetail shape is replicated, in negative, on the upper concave curved wall 91a of the main body 91, which is shaped to match the lower convex curved face 82a.

The slidable coupling between the above-mentioned upper concave curved wall 91a of the main body 91 and lower convex curved face 82a of the lower valve part 82 allows, as mentioned, the tilting of the first locking unit 8 relative to the second locking unit 9.

The dovetail shape of the slidable coupling just described advantageously allows a fluid movement and also the stable maintaining of the desired angular position of the first locking unit 8, obtained by the above-mentioned tilting movement.

As mentioned above, the tilting movement occurs along the tilting axis A3 which is transversal relative to the longitudinal axes of extension A1, A2 of the through holes 92, 93 made in the main body 91.

As clearly illustrated in Figure 3, that means that the tilting axis A3 is perpendicular to the direction D1 identified by the above-mentioned axes A1, A2 of the through holes 92, 94.

As illustrated in Figures 3 to 8, on the lower valve part 82 of the first locking unit 8, at its lower convex curved face 82a there is a graduated angular scale 11, whilst on the main body 91, at the upper concave curved wall 91a there is a reference mark 12, shown in Figure 8.

The graduated angular scale 11 and the reference mark 12 are configured to allow the operator acting on the clamp 2 to precisely measure the angle of inclination of the first and second locking units 8, 9 relative to each other.

As illustrated in Figures 6 to 9, the clamp 2 according to the invention comprises a friction pin 13 having an end 13a shaped to match a portion of the above-mentioned dovetail section of the lower convex curved face 82a.

The clamp 2 also comprises a thrust screw 14 designed to be positioned axially aligned with the above-mentioned pin 13 to push the pin 13 against the lower convex curved face 82a and to apply the relative frictional action on the lower valve part 82.

The thrust screw 14 has a control head 15 having a cavity advantageously with a hexagonal shape for engaging with a common hexagonal key.

As illustrated in Figures 6 and 7, in the main body 91 of the clamp 2 there is a cavity 16 for housing the above-mentioned friction pin 13 and thrust screw 14.

The housing cavity 16 is substantially cylindrical and has a central axis of extension A4 parallel to the tilting axis A3.

A portion of the housing cavity 16 is threaded in such a way as to engage by screwing/unscrewing the thrust screw 14 which, in its axial movement, applies a more or less marked force on the friction pin 13.

The above-mentioned friction pin 13, thrust screw 14 and housing cavities 16 define, for the clamp 2 respective stop means 17 configured for stably fixing the relative angular position of the first locking unit 8 and of the second locking unit 9, that is to say, stably fixing the desired angle of the bone stump connected to the clamp 2 relative to the remaining bone stump(s) connected to the remaining clamps 3 of the fixation device 1.

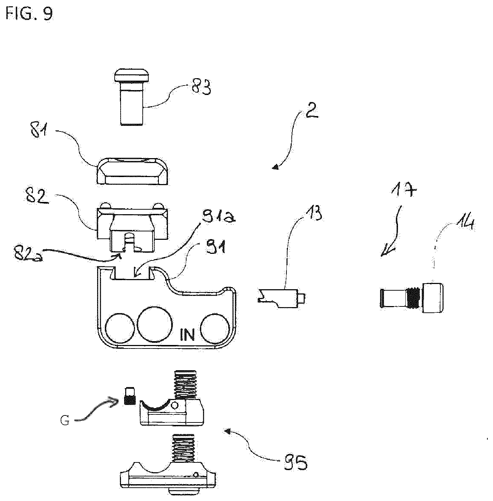

As illustrated in Figures 7 and 9, the clamp 2 comprises a grub screw G engaged by screwing in a respective threaded hole made in the main body 91 of the second locking unit 9.

The grub screw G advantageously prevents the undesired withdrawal of the first locking unit 8 from the second locking unit 9.

In other words, the grub screw G allows the tilting of the locking unit 8 but defines a shape impediment to its complete sliding relative to the second locking unit 9. The grub screw G can advantageously be operated by means of a common key with a hexagonal profile inserted in a suitable hole leading to a lower face 91b of the main body 91.

**In use,** once the surgeon has fixed the screws 4 of the clamp 2 to a relative bone stump and to the first locking unit 8, and the locking means 17 have been loosened, the stump can be rotated in position by the relative tilting of the first locking unit 8 relative to the second locking unit 8.

After reaching the desired angular position, which is easily displayed in terms of sexagesimal degrees on the graduated angular scale 11, the surgeon may stably fix the position by rotating in a clockwise direction the thrust screw 14 of the stop means 17. That way, the thrust screw 14 pushes the friction pin 13 against a portion of the lower convex curved face 82a of the lower valve part 82, applying to it a friction action that prevents it from moving relative to the main body 91.

According to alternative embodiments of the clamp according to the invention, the portions shaped to match for the sliding by tilting of the two first and second locking units may be inverted: that is to say, making the concave part on the first locking unit and the convex part on the main body of the second locking unit.

Advantageously, however, with such an arrangement more material should necessarily be used in the main body, thereby increasing the weight of the clamp and the fixation device. On the other hand, by forming the convex part in the first locking unit it is possible to minimise the overall dimensions, making the clamp advantageously limited in weight.

Advantageously, according to embodiments not illustrated of the clamp according to the invention, the tilting movement of the first locking unit 8 on the second locking unit 9 is controlled in a micrometric manner by means of the rotation of a suitable worm screw controlled by a respective key.

The clamp 1 according to the invention overcomes the above-mentioned drawbacks and brings important advantages.

A first advantage linked to the invention is due to the fact that it provides an extremely practical and versatile clamp by means of which it is possible to rotate the bone stump integral with it relative to a tilting axis transversal to the direction of extension of the fixation device.

A further advantage referred to the invention is due to the suitability of locking the relative movement between the lower valve part and the main body thanks to the particular arrangement and shape of the friction pin.

Moreover, the presence of a graduated angular scale and effective stop means allows extremely precise angular positions to be obtained and also allows them to be corrected and/or modified over time in view of the needs of the individual patient.

## Claims

1. A clamp of an external fixation device for the treatment of bone fractures, comprising:
- a first locking unit (8) designed to house and lock at least two bone screws (4),
- a second locking unit (9) designed to lock at least one connecting bar (5, 6), comprising at least one through hole (92, 94) for housing a connecting bar (5, 6), said hole (92, 94) having a longitudinal axis of extension (A1, A2), said first locking unit (8) tilting, relative to said second locking unit (9), according to a tilting axis (A3) transversal to said longitudinal axis of extension (A1, A2) of said through hole (92, 94), said first locking unit (8) having two valve parts (81, 82) respectively upper and lower, designed to couple for tightening between them said bone screws (4) housed in respective housings (10), said lower valve part (82) of said first locking unit (8) having a lower convex curved face (82a),
- stop means (17) configured for stably fixing the relative angular position of said first locking unit (8) and second locking unit (9), said clamp being **characterised in that** said stop means (17) comprise a friction pin (13) having one end shaped to match a portion of said lower convex curved face (82a), and a thrust screw (14) designed to be positioned axially aligned with respect to the friction pin (13) for pushing the friction pin (13) against said lower convex curved face (82a).

2. The clamp according to claim 1, **characterised in that** said tilting axis (A3) is positioned in the same direction as the directions of extension of said bone screws (4).

3. The clamp according to claim 2, **characterised in that** said tilting axis (A) is parallel to the direction of extension of at least one of said bone screws (4).

4. The clamp according to any one of the preceding claims, **characterised in that** said lower valve part (82) has a lower convex curved face (82a) configured to slidably engage with an upper concave curved wall (91a) of a main body (91) of said second locking unit (9), the relative movement of said lower convex curved face (82a) and upper concave curved wall (91a) defining the mutual tilting of said first and second locking units (8, 9).

5. The clamp according to claim 4, **characterised in that** said lower convex curved face (82a) and the upper concave curved wall (91a) comprise respective portions having dovetail sections, shaped to match each other to slidably engage in a stable fashion.

6. The clamp according to claim 4 or 5, **characterised in that** at one of said lower convex curved face (82a) and upper concave curved wall (91a) there is a graduated angular scale (11) and on the other of said lower convex curved face (82a) and upper concave curved wall (91a) there is a reference mark (12), said graduated angular scale (11) and reference mark (12) being configured to allow the measurement of the angle of mutual inclination of the first and second locking units (8, 9).

7. The clamp according to any of the preceding claims, **characterised in that** it comprises a cavity (16) for housing said friction pin (13) and thrust screw (14), said cavity (16) being formed in said main body (91) of said second locking unit (9) and having an axis of extension (A4) parallel to said tilting axis (A3).

8. An external fixation device for the treatment of bone fractures, comprising a clamp (2) according to any one of claims 1 to 7.

## Patentansprüche

1. Klemme einer externen Fixiervorrichtung zur Behandlung von Knochenbrüchen, umfassend:
- eine erste Verriegelungseinheit (8), die dazu ausgestaltet ist, mindestens zwei Knochenschrauben (4) aufzunehmen und zu verriegeln,
- eine zweite Verriegelungseinheit (9), die dazu ausgestaltet ist, mindestens eine Verbindungsstange (5, 6) zu verriegeln, die mindestens ein Durchgangsloch (92, 94) zur Aufnahme einer Verbindungsstange (5, 6) umfasst, wobei das Loch (92, 94) eine Längserstreckungsachse (A1, A2) aufweist, wobei sich die erste Verriegelungseinheit (8) relativ zur zweiten Verriegelungseinheit (9) gemäß einer Neigungsachse (A3) quer zur Längserstreckungsachse (A1, A2) des Durchgangslochs (92, 94) neigt, wobei die erste Verriegelungseinheit (8) zwei Ventilteile (81, 82) jeweils oben und unten aufweist, die dazu ausgestaltet sind, sich zu koppeln, um die in jeweiligen Gehäusen (10) aufgenommenen Knochenschrauben (4) zwischen ihnen anzuziehen, wobei der untere Ventilteil (82) der ersten Verriegelungseinheit (8) eine untere konvex gekrümmte Fläche (82a) aufweist,
- Anschlagmittel (17), die zum stabilen Fixieren der relativen Winkelposition der ersten Verriegelungseinheit (8) und der zweiten Verriegelungseinheit (9) ausgelegt sind, wobei die Klemme **dadurch gekennzeichnet ist, dass** die Anschlagmittel (17) einen Reibungsstift (13) mit einem Ende, das so geformt ist, dass es zu einem Abschnitt der unteren konvexen gekrümmten Fläche (82a) passt, und eine Druckschraube (14) umfassen, die so ausgestaltet ist, dass sie axial in Bezug auf den Reibungsstift (13) ausgerichtet ist, um den Reibungsstift (13) gegen die untere konvexe gekrümmte Fläche (82a) zu drücken.

2. Klemme nach Anspruch 1, **dadurch gekennzeichnet, dass** die Neigungsachse (A3) in der gleichen Richtung wie die Erstreckungsrichtungen der Knochenschrauben (4) positioniert ist.

3. Klemme nach Anspruch 2, **dadurch gekennzeichnet, dass** die Neigungsachse (A) parallel zur Erstreckungsrichtung mindestens einer der Knochenschrauben (4) verläuft.

4. Klemme nach einem der vorhergehenden Ansprüche, **dadurch gekennzeichnet, dass** der untere Ventilteil (82) eine untere konvex gekrümmte Fläche (82a) aufweist, die so ausgelegt ist, dass sie gleitend mit einer oberen konkav gekrümmten Wand (91a) eines Hauptkörpers (91) der zweiten Verriegelungseinheit (9) in Eingriff steht, wobei die Relativbewegung der unteren konvex gekrümmten Fläche (82a) und der oberen konkav gekrümmten Wand (91a) die gegenseitige Neigung der ersten und zweiten Verriegelungseinheit (8, 9) definiert.

5. Klemme nach Anspruch 4, **dadurch gekennzeichnet, dass** die untere konvex gekrümmte Fläche (82a) und die obere konkav gekrümmte Wand (91a) jeweilige Abschnitte mit Schwalbenschwanzabschnitten umfassen, die so geformt sind, dass sie zueinander passen, um in einer stabilen Weise gleitend in Eingriff zu treten.

6. Klemme nach Anspruch 4 oder 5, **dadurch gekennzeichnet, dass** an einer der unteren konvex gekrümmten Fläche (82a) und der oberen konkav gekrümmten Wand (91a) eine abgestufte Winkelskala (11) vorhanden ist und an der anderen der unteren konvex gekrümmten Fläche (82a) und der oberen konkav gekrümmten Wand (91a) eine Referenzmarkierung (12) vorhanden ist, wobei die abgestufte Winkelskala (11) und die Referenzmarkierung (12) ausgelegt sind, um die Messung des Winkels der gegenseitigen Neigung der ersten und zweiten Verriegelungseinheit (8, 9) zu ermöglichen.

7. Klemme nach einem der vorhergehenden Ansprüche, **dadurch gekennzeichnet, dass** sie einen Hohlraum (16) zur Aufnahme des Reibungsstiftes (13) und der Druckschraube (14) umfasst, wobei der Hohlraum (16) in dem Hauptkörper (91) der zweiten Verriegelungseinheit (9) ausgebildet ist und eine Erstreckungsachse (A4) parallel zu der Neigungsachse (A3) aufweist.

8. Externe Fixiervorrichtung zur Behandlung von Knochenbrüchen, umfassend eine Klemme (2) nach einem der Ansprüche 1 bis 7.

## Revendications

1. Pince d'un dispositif de fixation externe pour le traitement des fractures osseuses, comprenant :
- une première unité de verrouillage (8) conçue pour loger et verrouiller au moins deux vis à os (4),
- une seconde unité de verrouillage (9) conçue pour verrouiller au moins une barre de liaison (5, 6), comprenant au moins un trou traversant (92, 94) pour loger une barre de liaison (5, 6), ledit trou (92, 94) ayant un axe longitudinal d'extension (A1, A2), ladite première unité de verrouillage (8) basculant, par rapport à ladite seconde unité de verrouillage (9), selon un axe de basculement (A3) transversal audit axe longitudinal d'extension (A1, A2) dudit trou traversant (92, 94), ladite première unité de verrouillage (8) ayant deux parties de soupape (81, 82) respectivement supérieure et inférieure, conçues pour coupler de manière à serrer entre elles lesdites vis à os (4) logées dans des logements (10) respectifs, ladite partie de soupape inférieure (82) de ladite première unité de verrouillage (8) ayant une face incurvée convexe inférieure (82a),
- des moyens d'arrêt (17) configurés pour fixer de manière stable la position angulaire relative de ladite première unité de verrouillage (8) et de ladite seconde unité de verrouillage (9), ladite pince étant **caractérisée en ce que** lesdits moyens d'arrêt (17) comprennent une broche de friction (13) ayant une extrémité formée pour correspondre à une portion de ladite face incurvée convexe inférieure (82a), et une vis de poussée (14) conçue pour être positionnée axialement alignée par rapport à la broche de friction (13) pour pousser la broche de friction (13) contre ladite face incurvée convexe inférieure (82a).

2. Pince selon la revendication 1, **caractérisée en ce que** ledit axe d'inclinaison (A3) est positionné dans la même direction que les directions d'extension desdites vis à os (4).

3. Pince selon la revendication 2, **caractérisée en ce que** ledit axe de basculement (A) est parallèle à la direction d'extension d'au moins une desdites vis à os (4).

4. Pince selon l'une quelconque des revendications précédentes, **caractérisée en ce que** ladite partie de soupape inférieure (82) a une face incurvée convexe inférieure (82a) configurée pour s'engager de manière coulissante avec une paroi incurvée concave supérieure (91a) d'un corps principal (91) de ladite seconde unité de verrouillage (9), le mouvement relatif de ladite face incurvée convexe inférieure (82a) et de ladite paroi incurvée concave supérieure (91a) définissant le basculement mutuel desdites première et seconde unités de verrouillage (8, 9).

5. Pince selon la revendication 4, **caractérisée en ce que** ladite face incurvée convexe inférieure (82a) et la paroi incurvée concave supérieure (91a) comprennent des portions respectives ayant des sections en queue d'aronde, façonnées pour s'adapter l'une à l'autre pour s'engager de manière coulissante d'une façon stable.

6. Pince selon la revendication 4 ou 5, **caractérisée en ce qu'**au niveau de l'une de ladite face incurvée convexe inférieure (82a) et de ladite paroi incurvée concave supérieure (91a) il y a une échelle angulaire graduée (11) et sur l'autre de ladite face incurvée convexe inférieure (82a) et de ladite paroi incurvée concave supérieure (91a) il y a un repère de référence (12), ladite échelle angulaire graduée (11) et ledit repère de référence (12) étant configurés pour permettre la mesure de l'angle de basculement mutuel des première et seconde unités de verrouillage (8, 9).

7. Pince selon l'une quelconque des revendications précédentes, **caractérisée en ce qu'**elle comprend une cavité (16) pour loger ladite broche de friction (13) et ladite vis de poussée (14), ladite cavité (16) étant formée dans ledit corps principal (91) de ladite seconde unité de verrouillage (9) et ayant un axe d'extension (A4) parallèle audit axe de basculement (A3).

8. Dispositif de fixation externe pour le traitement des fractures osseuses, comprenant une pince (2) selon l'une quelconque des revendications 1 à 7.
